# EUROPEAN PATENT APPLICATION

(11) **EP 1 174 148 A1**
(43) Date of publication of application: **23.01.2002**
(21) Application number: 00922896.6
(22) Date of filing: 27.04.2000
(51) Int. Cl.: A61K 39/395, A61K 9/08, A61K 9/19, A61K 47/26, A61K 47/34, C07K 16/36

(54) **PARENTERAL MEDICINAL COMPOSITION CONTAINING HUMANIZED MONOCLONAL ANTIBODY FRAGMENT AND METHOD FOR STABILIZING THE SAME**

(30) Priority: 28.04.1999 JP 12142499
(71) Applicant: YAMANOUCHI PHARMACEUTICAL CO. LTD., Tokyo 103-8411 (JP)
(72) Inventor: OKADA, Akira Yamanouchi Pharmaceutical Co., Ltd., Yaizu-shi, Shizuoka 425-0072 (JP); KOBAYASHI, Mitsugu, Yamanouchi Pharm. Co., Ltd., Tokyo 174-8612 (JP); MORI, Atsuhide Yamanouchi Pharmaceutical Co., Ltd., Yaizu-shi, Shizuoka 425-0072 (JP)
(74) Representative: Geering, Keith Edwin
(86) International application number: JP0002784
(87) International publication number: WO0066160

(57) **Abstract**

This invention relates to a parenteral pharmaceutical composition which comprises a humanized monoclonal antibody fragment, a nonionic surface active agent and saccharides, wherein its pH is weakly acidic. The invention also relates to a method for the stabilization of a humanized antibody fragment, which comprises formulating a nonionic surface active agent and saccharides and adjusting the pH to a weakly acidic level.

According to the invention, a stable parenteral pharmaceutical composition or parenteral pharmaceutical preparation can be provided, which comprises a humanized monoclonal antibody fragment and has no using limitations such as cold place preservation avoiding freezing, transfer and handling avoiding shaking, particle removing operation by a filter when used and the like.

## Description

### Technical Field

This invention relates to a stable parenteral pharmaceutical composition which comprises a humanized monoclonal antibody fragment. Particularly, the invention relates to a stable parenteral pharmaceutical composition which comprises Fab fragment of a humanized monoclonal antibody for a fibrinogen receptor of a human platelet membrane glycoprotein GPIIb/IIIa. The invention also relates to a method for the stabilization of a humanized monoclonal antibody fragment, which comprises formulating a nonionic surface active agent and saccharides and adjusting the pH to a weakly acidic level.

### Background Art

Since the proposal of a method for the mass production of monoclonal antibodies by means of genetic engineering, monoclonal antibodies have been broadly used in the field of medicaments.

Recently, Centocor in the United States has developed "ReoPro (trade name)" comprised of a human-mouse chimeric monoclonal antibody fragment and is providing it as a platelet aggregation inhibitor in the clinical field. This pharmaceutical preparation is a liquid preparation of pH 7.2 containing 2 mg/ml of a chimeric monoclonal antibody fragment, 0.01 M of sodium phosphate, 0.15 M of sodium chloride and 0.001% of polysorbate 80. Since this preparation is a liquid preparation, it is not necessary to use it by dissolving in distilled water for injection or the like prior to use as in the case of freeze-dried preparations. However, this preparation has some limitations in handling it, e.g., to store it in a cold place (2 to 8°) while avoiding freezing, to avoid shaking, and to remove particles with a filter prior to its administration. Because of such limitations, it is considered that this preparation is a preparation which is difficult to handle.

Accordingly, great concern has been directed toward the development of a pharmaceutical preparation having none of such limitations in handling.

The object of the invention is to provide a stable parenteral pharmaceutical composition or parenteral pharmaceutical preparation which comprises a humanized monoclonal antibody fragment and has no using limitations such as cold place preservation avoiding freezing, transfer and handling avoiding shaking, particle removing operation by a filter when used and the like, and a method for the stabilization of the humanized monoclonal antibody fragment.

### Disclosure of the Invention

Under such a situation, the present inventors have conducted intensive studies and when an Fab fragment was obtained by papain digestion of a humanized C4G1 antibody produced by the method described in International Publication WO 93/13133 (corresponding U.S. Patent 5777085, corresponding European Patent EP 619324) and then the Fab fragment was purified in accordance with the description in said specification, it was found that the thus obtained humanized C4G1 Fab fragment can be stabilized by adding a nonionic surface active agent and saccharides to the purified Fab fragment and adjusting pH of the mixture to a weakly acidic level with a buffer, and the invention has been accomplished as a result of further continued studies. That is, the invention relates to a parenteral pharmaceutical preparation which comprises a humanized monoclonal antibody fragment, a nonionic surface active agent and saccharides, wherein the pH is weakly acidic. Particularly, the invention relates to a parenteral pharmaceutical preparation which comprises a humanized monoclonal antibody fragment, as Fab fragment of a humanized monoclonal antibody for a fibrinogen receptor of a human platelet membrane glycoprotein GPIIb/IIIa, a nonionic surface active agent and saccharides, wherein the pH is weakly acidic. The invention also relates to a method for the stabilization of the humanized monoclonal antibody fragment, which comprises formulating a nonionic surface active agent and saccharides and adjusting the pH to a weakly acidic level.

The humanized monoclonal antibody fragment to be used in the invention is not particularly limited with the proviso that it generally has a therapeutically effective pharmacological action as a medicament. For example, it may be any one of fragments prepared by making humanized monoclonal antibodies described in JP-A-62-296890 (corresponding U.S. Patent 5225539, corresponding European Patent 239400; the term "JP-A" as used herein means an "unexamined published Japanese patent application"), International Publication WO 90/7861 (corresponding U.S. Patent 5693761, corresponding European Patent 451216) and the like into fragments by a method well known in said technical field (e.g., a chemical technique or enzymatic technique), and their molecular weight is not particularly limited, too. Also, it may be a fragment described, e.g., in WO 93/13133, which is directly produced by genetic engineering techniques. Preferred is a fragment having a platelet aggregation inhibition action. As such a fragment, e.g., an Fab fragment of a humanized monoclonal antibody for a fibrinogen receptor of a human platelet membrane glycoprotein GPIIb/IIIa can be cited. Illustratively, a humanized C4G1 Fab fragment, prepared by digesting the humanized C4G1 Fab antibody produced by the method described in International Publication WO 93/13133 with a proteolytic enzyme (e.g., papain) using a method well known in said technical field, thereby obtaining an Fab fragment, and then purifying the fragment in accordance with the description in said specification, is more desirable as such an Fab fragment (cf. the drawings which will be described later).

According to the invention, the amount of the Fab fragment is not particularly limited, with the proviso that it is an amount capable of generally exerting a therapeutically effective pharmacological action as a medicament, but is preferably from 2 mg to 100 mg, more preferably from 5 mg to 50 mg.

According to the invention, the concentration of the Fab fragment is not particularly limited, with the proviso that it is generally within such a range that a parenteral pharmaceutical composition can be provided, but is preferably from 0.01 mg/ml to 10 mg/ml, more preferably from 0.1 to 8 mg/ml. When the concentration is lower than 0.01 mg/ml, there will be a case in which its provision as a pharmaceutical preparation is difficult in reality, because the preparation becomes large in size in order to keep the concentration for expressing effective pharmacological action. Also, when the concentration is higher than 10 mg/ml, it becomes close to the saturation solubility of the fragment, thus posing a possibility of generating aggregates during preservation.

The nonionic surface active agent to be used in the invention is not particularly limited with the proviso that it is generally pharmaceutically acceptable. In this case, the nonionic surface active agent means a surface active agent which does not show ionic property, such as a polyalkylene glycol ether of an aliphatic alcohol, a polyalkylene glycol ether of an alkyl phenol or the like. For example, polysorbate 80, polysorbate 20 and the like can be cited, of which preferred is polysorbate 80 and more preferred is plant-originated polysorbate 80. The nonionic surface active agent of the invention can be formulated alone or as a combination of two or more species.

According to the invention, the concentration of the nonionic surface active agent is not particularly limited, with the proviso that it is generally within such a range that a parenteral pharmaceutical composition can be provided, but is preferably within a range of from about 1 x 10⁻⁵% by weight to 1% by weight, more preferably from 0.0001% by weight to 0.1% by weight, in the solution. When the concentration is lower than 1 x 10⁻⁵% by weight, it will pose a possibility of generating aggregates by shaking. In this connection, the nonionic surface active agent of the invention is added mainly to inhibit formation of aggregates.

The saccharides to be used in the invention are not particularly limited with the proviso that they are generally pharmaceutically acceptable. Examples of such saccharides include glucose, xylose, galactose, fructose and the like monosaccharides, lactose, maltose, purified sucrose, sucrose and the like disaccharides and mannitol, sorbitol, xylitol and the like sugar alcohols. Preferred are purified sucrose and/or mannitol. The saccharides of the invention can be formulated alone or as a combination of two or more. In this connection, the saccharides of the invention are added mainly to stabilize the humanized monoclonal antibody and have functions, e.g., to adjust osmotic pressure of the pharmaceutical preparation and to keep matrix components amorphous so that re-dissolution of the preparation becomes easy when it is freeze-dried.

According to the invention, the concentration of the saccharides is not particularly limited, with the proviso that it is generally within such a range that a parenteral pharmaceutical composition can be provided, but is preferably from 0.01% by weight to 50% by weight, more preferably from 0.1% by weight to 10% by weight. When the concentration is lower than 0.1% by weight, it will pose a possibility of generating aggregates by shaking. Also, when it is higher than 50% by weight, there is a possibility that saccharides and the like are precipitated. In addition, when the concentration is within this range, the saccharides also exert the effect as a bulking agent when made into a freeze-dried preparation.

According to the invention, pH of the parenteral pharmaceutical composition or preparation is not particularly limited when it is adjusted to a weakly acidic level by a known method. Preferably, the pH is adjusted to approximately from 4 to 6 by formulating a substance having the action to buffer at a weakly acidic level (to be referred to as a buffer hereinafter). According to the invention, the term weakly acidic means a pH value of approximately from 4 to 6. When the pH is from neutral to alkaline, stability of the preparation is considerably spoiled, such as increase in impurities, increase in aggregates and the like. Also, when the preparation is strongly acidic, it is desirable to avoid its use as injections due to pain and the like. Examples of the buffer include a phosphate buffer (e.g., phosphoric acid-disodium hydrogenphosphate buffer), a citrate buffer (e.g., citric acid-sodium hydroxide), an acetate buffer (e.g., acetic acid-sodium acetate), a tartarate buffer (e.g., tartaric acid-sodium hydroxide), a malate buffer (e.g., malic acid-sodium hydroxide), a histidine buffer (e.g., histidine-hydrochloric acid), an arginine buffer (e.g., arginine-hydrochloric acid) and the like. When the parenteral pharmaceutical composition is used as injections, their sodium salts are preferable, and sodium phosphate buffer and/or sodium citrate buffer is more preferable. These buffers of the invention may be used alone or as a combination of two or more.

According to the invention, the concentration of the buffer is not particularly limited, with the proviso that it is generally within such a range that a parenteral pharmaceutical composition can be provided, but is preferably 1 mM or more, preferably from 1 mM to 500 mM. When the concentration is lower than 1 mM, it is difficult to keep the pH stably due to too weak buffer action. Since osmotic pressure becomes high when it exceeds 500 mM, the composition may be used by diluting with, e.g., distilled water for injection prior to use, but it is desirable that the concentration is from 1 mM to 500 mM when the parenteral pharmaceutical composition or preparation is directly used without diluting prior to use.

The parenteral pharmaceutical preparation obtained by carrying out the invention is not particularly limited, with the proviso that it is in a generally pharmaceutically acceptable dosage form, but it is preferably in the form of aseptic preparations such as aqueous injections, nonaqueous injections, injections to be dissolved prior to use (e.g., a preparation powdered by freeze drying method) and the like. In this connection, as the freeze-drying conditions, known conditions can be set optionally.

The parenteral pharmaceutical preparation of the invention is preserved generally in sealed containers as aseptic preparations such as aqueous injections, nonaqueous injections, injections to be dissolved prior to use (e.g., a preparation powdered by freeze drying method) and the like, and it is desirable that the space is under an oxygen-reduced atmosphere. In this case, the term "under an oxygen-reduced atmosphere" means an atmosphere in which oxygen in the air is artificially reduced. For this, it is desirable that the atmosphere in the sealed container is replaced, e.g., with an inert gas (e.g., nitrogen gas). More preferred is nitrogen gas. Also, the gas replacing ratio is preferably 90% or more, more preferably 95% or more.

Regarding the production method of the parenteral pharmaceutical preparation of the invention, a conventionally known method can be employed, and its example includes a method in which the Fab fragment produced by the method described in International Publication WO 93/13133 is mixed with and dissolved in solution containing additives such as a nonionic surface active agent, saccharides and the like, and the resulting solution is adjusted by mixing with a dilution buffer solution adjusted to result in the final concentration. In this case, since the final composition of the Fab fragment solution is influenced by the method of the final step of purification, the final concentration of each component can be optionally set by liquid composition-exchanging or concentrating the Fab fragment solution by diafiltration or the like method.

The parenteral pharmaceutical composition of the invention can be mixed with pharmaceutical additives generally added to parenteral pharmaceutical compositions (e.g., a solubilizing agent, a preservative, a stabilizing agent, an emulsifying agent, a soothing agent, a tonicity agent, a buffer agent, a bulking agent, a coloring agent and a thickening agent). For example, cyclodextrins and the like can be cited as the solubilizing agent. Methyl p-benzoate and the like can be cited as the preservative. Lecithin and the like can be cited as the emulsifying agent. Benzyl alcohol and the like can be cited as the soothing agent. Sodium chloride and the like can be cited as the tonicity agent. Maltose and the like can be cited as the bulking agent. Hyaluronic acid and the like can be cited as the thickening agent.

### Brief Description of the Drawings

Fig. 1 briefly illustrates the production process of a fragment (10) of a humanized monoclonal antibody.
Fig. 2 illustrates the action mechanism of a platelet aggregation inhibiting drug (platelet aggregation inhibitor).

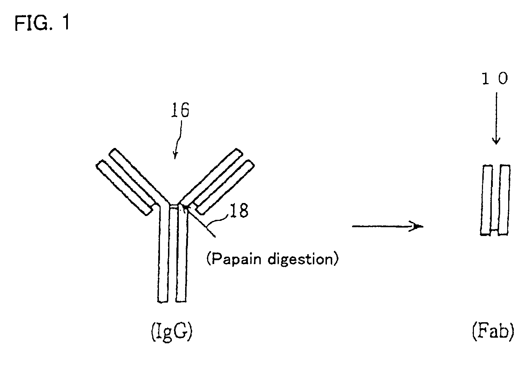

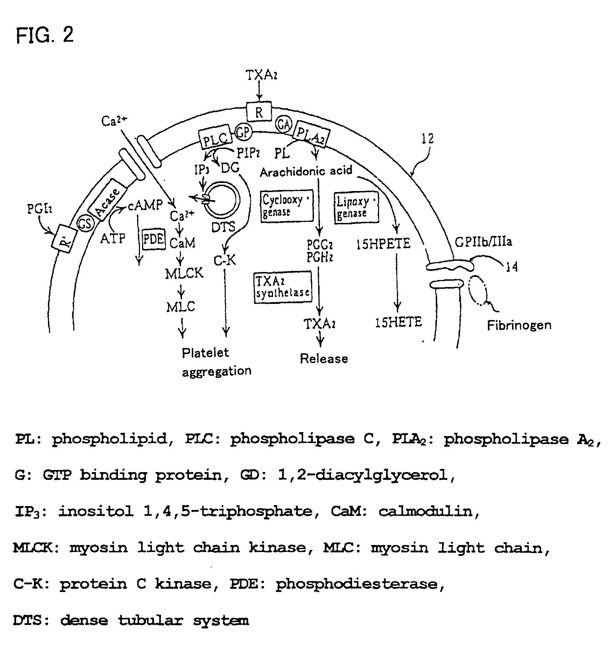

As shown in Figs. 1 and 2, the humanized monoclonal antibody fragment (10) to be used in the invention is produced by a process in which a monoclonal antibody for a fibrinogen receptor (14) of a glycoprotein GPIIb/IIIa existing on the surface of human platelet (12) is humanized (16) and then made into an Fab fragment by papain treatment (18).

### (Description of the Reference Numbers and Signs)

10: Fragment, 12: human platelet, 14: fibrinogen receptor, 16: humanized antibody, 18: papain treatment

### Best Mode for Carrying Out the Invention

The following describes the invention further illustratively with reference to examples which, however, do not limit the scope of the invention.

### [Reference Example]

An Fab fragment obtained by the following method was used as the humanized monoclonal antibody fragment. That is, a humanized C4G1 antibody obtained by the method described in International Publication WO 93/13133 (Example) was digested by a papain treatment to prepare an Fab fragment, and then the Fab fragment was purified in accordance with the description in said specification, thereby obtaining a humanized C4G1 Fab fragment (to be referred simply to as "Fab fragment" hereinafter).

### [Test Method 1] Titer measurement by binding-inhibition activity

Titers relative to a standard Fab fragment preparation are measured by allowing a biotinylated fibrinogen solution and an Fab fragment solution to react competitively with a GPIIb/IIIa-immobilized plate and developing a color with an avidin peroxidase solution.

### [Test Method 2] Determination of high molecular impurities by a high performance liquid chromatography

A 20 µl portion of a solution containing 1 mg of the Fab fragment is tested by a liquid chromatography under the following conditions. Peak areas are measured by an automatic integration method to calculate area percentages of peak areas other than that of the Fab fragment.
Detector: Ultraviolet absorption photometer
Column: Dextran-covalent bonded agarose of 13 µm in particle size is packed in a glass tube having an inner diameter of about 10 mm and a length of about 30 cm.
Column temperature: Constant temperature at around 25°C
Mobile phase: A 3.12 g portion of sodium dihydrogenphosphate and 11.7 g of sodium chloride are dissolved in 900 ml of water, and the solution is adjusted to pH 7.0 by adding 8 N sodium hydroxide solution and then filled up to 1,000 ml.
Liquid quantity: Each sample is adjusted to such an amount that retention time of the Fab fragment peak becomes about 38 minutes.

### [Test Method 3] Verification of appearance and aggregates by visual observation

The appearance of and the amounts of aggregates in liquid samples are compared by visual observation under an illumination intensity of from 2,000 lux to 5,000 lux.

### [Inventive Examples 1 to 3] [Comparative Examples 1 to 5]

A purified preparation of the Fab fragment having a concentration of about 1 mg/ml was subjected to diafiltration to change it to an Fab fragment aqueous solution having a concentration of from 3 to 6 mg/ml to be used as an Fab fragment bulk drug. Also, separately, various buffer solutions shown in Table 1 were prepared using respective components in such amounts that their concentrations at the time of final fill up became 2 mg/ml as the Fab fragment concentration, 10 mM as the buffer concentration, 0.01% by weight as the polysorbate 80 concentration and 5% by weight as the purified sucrose concentration, respectively, and mixed with the above bulk drug, thereby obtaining formulated solutions. Each of these formulated solutions was subjected to aseptic filtration and then dispensed in 3 to 5 ml portions into previously sterilized vials under aseptic environment, the head space in each vial was replaced with nitrogen by repeating suction and de-suction in a lyophilization chamber, and then each of the resulting vials was sealed with a stopper to obtain pharmaceutical preparations of the invention. The inventive preparations and comparative preparations were stored at 40°C and 60°C to compare their stability.

The test results are shown in Table 1. As is evident from Table 1, high molecular weight impurities were increased and the samples became opaque under the severe (high temperature) condition for the pH values of Comparative Examples, while reduction of titers was not observed by the pH values of the invention. Accordingly, it can be said that the pharmaceutical preparations of the invention are preparations having markedly high stability.

**(Table 1)**

| | Buffer agent | pH | Results Upper column: titer (%) Middle column: property Lower column: high molecular impurities (%) | | |
|---|---|---|---|---|---|
| | | | Initial stage | 40°C 1 month | 60°C 4 weeks |
| Inventive Ex. 1 | Sodium phosphate | 4.90 | 100 | 100 | 72 |
| | | | colorless | - | colorless |
| | | | 0.02 | 0.07 | 1.51 |
| Inventive Ex. 2 | Sodium phosphate | 5.95 | 100 | 100 | 81 |
| | | | colorless | - | colorless |
| | | | 0.03 | 0.10 | 1.08 |
| Inventive Ex. 3 | Sodium citrate | 5.21 | 100 | 81 | 77 |
| | | | colorless | - | colorless |
| | | | 0.06 | 0.04 | 2.40 |
| Comparative Ex. 1 | Sodium phosphate | 7.03 | 100 | 91 | 59 |
| | | | colorless | - | opaque |
| | | | 0.03 | 0.17 | 1.69 |
| Comparative Ex. 2 | Sodium phosphate | 7.85 | 100 | - | - |
| | | | colorless | - | opaque |
| | | | 0.04 | 0.39 | 10.72 |
| Comparative Ex. 3 | Sodium citrate | 7.14 | 100 | 68 | 56 |
| | | | colorless | - | opaque |
| | | | 0.13 | 0.13 | 2.78 |
| Comparative Ex. 4 | Sodium phosphate | 9.04 | 100 | - | - |
| | | | colorless | - | opaque |
| | | | 0.04 | 1.48 | 62.06 |
| Comparative Ex. 5 | Tris-HCl | 7.16 | 100 | 83 | 80 |
| | | | colorless | - | opaque |
| | | | 0.07 | 1.62 | 0.64 |

### [Inventive Examples 4 to 9] [Comparative Examples 6 and 7 (polysorbate 80)]

A purified preparation of the Fab fragment having a concentration of about 1 mg/ml was subjected to diafiltration to change it to an Fab fragment aqueous solution having a concentration of from 3 to 6 mg/ml to be used as an Fab fragment bulk drug. Also, separately, a buffer solution was prepared using respective components in such amounts that their concentrations at the time of final fill up became 2 mg/ml as the Fab fragment concentration, 10 mM as the sodium phosphate concentration and 5% by weight as the purified sucrose concentration, respectively, and mixed with the above bulk drug, thereby obtaining formulated solutions. Each of these formulated solutions was subjected to aseptic filtration and then dispensed in 3 to 5 ml portions into previously sterilized vials under aseptic environment, the head space in each vial was replaced with nitrogen by repeating suction and de-suction in a lyophilization chamber, and then each of the resulting vials was sealed with a stopper to obtain pharmaceutical preparations of the invention. The inventive preparations and comparative preparations were shaken at 200 rpm for 10 minutes to verify the presence or absence of aggregate formations.

The test results are shown in Table 2. As is evident from Table 2, aggregate formations can be considerably reduced by the addition of 1 x 10⁻⁵% by weight or more of polysorbate 80 even when shaking or the like physical stress was applied.

**(Table 2)**

| | Ionic surface active agent | | Visual confirmation of aggregates |
|---|---|---|---|
| | Kind | Concentration (% by weight) | |
| Inventive Ex. 4 | polysorbate 80 | 1 | no |
| Inventive Ex. 5 | polysorbate 80 | 0.1 | no |
| Inventive Ex. 6 | polysorbate 80 | 0.01 | no |
| Inventive Ex. 7 | polysorbate 80 | 0.001 | no |
| Inventive Ex. 8 | polysorbate 80 | 0.0001 | no |
| Inventive Ex. 9 | polysorbate 80 | 0.00001 | no |
| Comparative Ex. 6 | polysorbate 80 | 0.000001 | yes |
| Comparative Ex. 7 | polysorbate 80 | 0 | yes |

### [Inventive Example 10] [Comparative Example 8 (saccharides)]

A purified preparation of the Fab fragment having a concentration of about 1 mg/ml was subjected to diafiltration to change it to an Fab fragment aqueous solution having a concentration of from 3 to 6 mg/ml to be used as an Fab fragment bulk drug. Also, separately, a buffer solution was prepared using respective components in such amounts that their concentrations at the time of final fill up became 2 mg/ml as the Fab fragment concentration, 10 mM as the sodium phosphate concentration and 0.01% by weight as the polysorbate 80 concentration, respectively, and mixed with the above bulk drug, thereby obtaining formulated solutions. Each of these preparation solutions was subjected to aseptic filtration and then dispensed in 3 to 5 ml portions into previously sterilized vials under aseptic environment, the head space in each vial was replaced with nitrogen by repeating suction and de-suction in a lyophilization chamber, and then each of the resulting vials was sealed with a stopper to obtain pharmaceutical preparations of the invention. The inventive preparation and comparative preparation were stored at 60°C for 4 weeks to verify the presence or absence of aggregate formations.

The test results are shown in Table 3. As is evident from Table 3, it was able to prevent generation of aggregates during preservation by the inventive pharmaceutical preparation of the invention to which purified sucrose was added.

**(Table 3)**

| | Saccharide | | Visual confirmation of aggregates |
|---|---|---|---|
| | Kind | Concentration (% by weight) | |
| Inventive Ex. 10 | purified sucrose | 5 | no |
| Comparative Ex. 8 | absent | 0 | insoluble foreign matter |

### [Inventive Example 11]

A purified preparation of the Fab fragment having a concentration of about 1 mg/ml was subjected to diafiltration to change it to an Fab fragment aqueous solution having a concentration of from 3 to 6 mg/ml to be used as an Fab fragment bulk drug. Also, separately, a buffer solution was prepared using respective components in such amounts that their concentrations at the time of final fill up became 2 mg/ml as the Fab fragment concentration, 10 mM as the sodium phosphate concentration, 0.01% by weight as the polysorbate 80 concentration and 5% by weight as the purified sucrose concentration, respectively, and mixed with the above bulk drug, thereby obtaining a preparation solution. This preparation solution was subjected to aseptic filtration and then dispensed in 3 to 5 ml portions into previously sterilized vials under aseptic environment, the head space in each vial was replaced with nitrogen by 95% by repeating suction and de-suction in a lyophilization chamber, and then each of the resulting vials was sealed with a stopper to obtain a pharmaceutical preparation. This was stored at 40°C and 60°C to compare the stability during preservation.

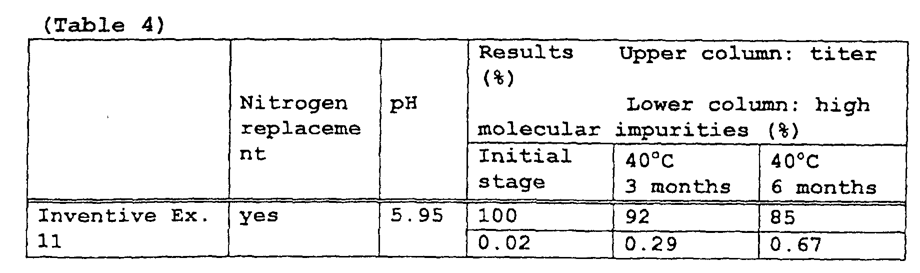

### Industrial Applicability

The parenteral pharmaceutical composition or preparation of the invention exerts excellent effects under a liquid state or a freeze-dried state, namely, it shows excellent preservation stability, it can be stored at room temperature, it inhibits aggregate formations so that it does not require particle removing step by a filter, and it can be used conveniently.

## Claims

1. A parenteral pharmaceutical composition which comprises a humanized monoclonal antibody fragment, a nonionic surface active agent and saccharides, wherein the pH is weakly acidic.

2. The parenteral pharmaceutical composition according to claim 1, wherein the humanized monoclonal antibody fragment has an action to inhibit aggregation of human platelets.

3. The parenteral pharmaceutical composition according to claim 1 or 2, wherein the humanized monoclonal antibody fragment is an Fab fragment of a humanized monoclonal antibody for a fibrinogen receptor of a human platelet membrane glycoprotein GPIIb/IIIa.

4. The parenteral pharmaceutical composition according to claim 3, wherein the concentration of the Fab fragment is from 0.01 mg/ml to 10 mg/ml.

5. The parenteral pharmaceutical composition according to any one of claims 1 to 4, wherein it is further blended with a substance having an action to buffer weakly acidic to adjust the pH value approximately 4 to 6.

6. The parenteral pharmaceutical composition according to claim 5, wherein the substance having an action to buffer weakly acidic is sodium phosphate and/or sodium citrate.

7. The parenteral pharmaceutical composition according to any one of claims 1 to 6, wherein the concentration of the substance having an action to buffer weakly acidic is from 1 mM to 500 mM.

8. The parenteral pharmaceutical composition according to any one of claims 1 to 7, wherein the nonionic surface active agent is polysorbate 80.

9. The parenteral pharmaceutical composition according to any one of claims 1 to 8, wherein the concentration of the nonionic surface active agent is from 1 x 10⁻⁵% by weight to 1% by weight.

10. The parenteral pharmaceutical composition according to any one of claims 1 to 9, wherein the saccharides are purified sucrose and/or mannitol.

11. The parenteral pharmaceutical composition according to any one of claims 1 to 10, wherein the concentration of the saccharides is from 0.01% by weight to 50% by weight.

12. A parenteral pharmaceutical preparation which is produced by freeze-drying the parenteral pharmaceutical composition of any one of claims 1 to 11.

13. A parenteral pharmaceutical preparation in which the parenteral pharmaceutical composition of any one of claims 1 to 12 is preserved in an oxygen-reduced atmosphere.

14. A method for stabilizing a humanized monoclonal antibody fragment, which comprises formulating a nonionic surface active agent and saccharides and adjusting the pH to a weakly acidic level.

15. The method for stabilizing a humanized monoclonal antibody fragment according to claim 14, wherein it is further preserved in an oxygen-reduced atmosphere.
